Europäisches Patentamt
European Patent Office
Office européen des brevets

(11) Publication number: 0 183 848 B1

# EUROPEAN PATENT SPECIFICATION

(45) Date of publication of patent specification: 12.09.90

(51) Int. Cl.$^5$: C 07 D 487/04, A 61 K 31/505

(21) Application number: 85901549.7

(22) Date of filing: 07.03.85

(86) International application number: PCT/JP85/00115

(87) International publication number: WO 85/04172 26.09.85 Gazette 85/21

## (54) POLYAZAHETEROCYCLIC DERIVATIVES, PROCESS FOR THEIR PREPARATION, AND PHARMACEUTICAL COMPOSITION.

(30) Priority: 12.03.84 JP 47357/84

(43) Date of publication of application: 11.06.86 Bulletin 86/24

(45) Publication of the grant of the patent: 12.09.90 Bulletin 90/37

(84) Designated Contracting States: AT BE CH DE FR GB LI NL SE

(56) References cited:
JP-A-4 738 992
JP-A-4 864 097
JP-A-5 076 086
JP-A-50 129 592
JP-A-54 160 745

(73) Proprietor: Yoshitomi Pharmaceutical Industries, Ltd.
35 Hiranomachi 3-chome Higashi-ku
Osaka-shi Osaka 541 (JP)

(72) Inventor: TSUDA, Yoshinao
2884-24, Higashisayamagaoka 3-chome
Tokorozawa-shi
Saitama 359 (JP)
Inventor: MISHINA, Tadashi
4-17-601, Ohgimachiya 1-chome Iruma-shi
Saitama 358 (JP)
Inventor: OBATA, Minoru
4-36, Okidaimachi 1-chome Nakatsu-shi
Oita 871 (JP)
Inventor: ARAKI, Kazuhiko 1212-4, Aza-Azema
Oaza-Kamiikenaga
Nakatsu-shi Oita 871-01 (JP)
Inventor: INUI, Jun
1129-78, Oaza-Noda Iruma-shi
Saitama 358 (JP)
Inventor: NAKAMURA, Tadao
468-1, Shimomiyanaga 2-chome Nakatsu-shi
Oita 871 (JP)

(74) Representative: von Kreisler, Alek, Dipl.-Chem. et al
Deichmannhaus am Hauptbahnhof
D-5000 Köln 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 183 848 B1

Courier Press, Leamington Spa, England.

## Description

A polyazaheterocycle derivative, a method of preparing said compound and a pharmaceutical composition.

Background Art

EP—A 68302 discloses imidazo[1,2-a]pyrimidine derivatives represented by, for example, 8-(2,6-dichlorophenyl)-2,3,5,8-tetrahydro-imidazo[1,2-a]pyrimidine which are useful as analgesics, hypotensives and bradycardics.

Field of the Invention and Disclosure of the Invention

The present invention relates to novel and pharmaceutically useful polyazaheterocycle derivatives methods of preparing said compounds and pharmaceutical compositions containing said compounds.

The polyazaheterocycle derivative has the formula:

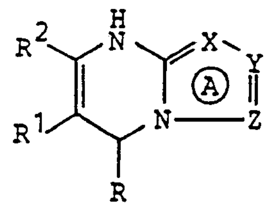

(I)

wherein R is a hydrogen atom, a $C_{1-5}$ alkyl group (e.g. methyl, ethyl, propyl, isopropyl, butyl, isobutyl, sec-butyl, tert-butyl or pentyl), a $C_{3-5}$ cycloalkyl group (e.g. cyclopropyl, cyclobutyl, cyclopentyl or cyclohexyl), a furyl group, a thienyl group, a pyridyl group, or a phenyl group which may be optionally substituted by one or two substituents selected from the group consisting of a halogen atom (fluorine, chlorine, bromine or iodine), a $C_{1-4}$ alkyl group (e.g. methyl, ethyl, propyl, isopropyl or butyl), a trifluoromethyl group, a $C_{1-4}$ alkoxy group (e.g. methoxy, ethoxy, propoxy or butoxy), a phenyl-$C_{1-3}$ alkyl-oxy group (e.g. benzyloxy, phenylethoxxy or phenylpropoxy), a $C_{1-4}$ alkyl-thio group (e.g. methylthio, ethylthio, propylthio or butylthio), a phenyl-$C_{1-3}$ alkyl-thio group (e.g. benzylthio, phenylethylthio or phenylpropylthio), a $C_{2-4}$ alkanoyl group (e.g. acetyl, propionyl or butyryl), a benzoyl group, a hydroxyl group, an amino group, a nitro group and a cyano group;

$R^1$ is a hydrogen atom; a cyano group; a carbamoyl group; a carboxyl group; a $C_{1-4}$ alkoxy-carbonyl group (e.g. methoxycarbonyl, ethoxycarbonyl, propoxycarbonyl or tert-butoxycarbonyl) which may be optionally substituted in the alkoxy moiety by one or two substituents selected from the group consisting of a $C_{1-4}$ alkoxy group (e.g. methoxy, ethoxy, propoxy or isopropoxy), a cyano group, a group represented by the formula: —N($Ra^1$)($Rb^1$) [wherein each of $Ra^1$ and $Rb^1$ is a $C_{1-4}$ alkyl group (e.g. methyl, ethyl, propyl, isopropyl, butyl or tert-butyl) or a phenyl-$C_{1-3}$ alkyl group (e.g. benzyl, phenylether or phenylpropyl), or $Ra^1$ and $Rb^1$ together with the adjacent nitrogen atom form a 5- to 7-membered heterocycle selected from the group consisting of pyrrolidine, piperidine, morpholine, piperazine, N-methylpiperazine, N-(2-hydroxyethyl)piperazine, azepine and diazepine], a nitro group, a phenyl group and a heteroaryl group selected from the group consisting of thienyl, furyl and pyridyl; or a $C_{2-6}$ alkanoyl group selected from the group consisting of acetyl, propionyl, butyryl, isobutyryl, valeryl, isovaleryl, pivaloyl and hexanoyl;

$R^2$ is a $C_{1-5}$ alkyl group (e.g. methyl, ethyl, propyl, isopropyl, butyl, isobutyl, sec-butyl, tert-butyl or pentyl), a hydroxy-$C_{1-4}$ alkyl group (e.g. hydroxymethyl, hydroxyethyl, hydroxypropyl or hydroxybutyl), an acetoxy-$C_{1-4}$ alkyl group (e.g. acetoxymethyl, acetoxyethyl, acetoxypropyl or acetoxybutyl), a halo-$C_{1-4}$ alkyl group (e.g. fluoromethyl, chloromethyl, bromomethyl, iodomethyl, fluoroethyl, chloroethyl, fluoropropyl, chloropropyl, fluorobutyl or chlorobutyl), a dihalo-$C_{1-4}$ alkyl group (e.g. difluoromethyl, dichloromethyl, dibromomethyl, difluoroethyl, dichloroethyl, difluoropropyl, dichloropropyl, difluorobutyl or dichlorobutyl), a trifluoromethyl group, a $C_{1-4}$ alkoxy-$C_{1-4}$ alkyl group (e.g. methoxymethyl, ethoxymethyl, propoxymethyl, butoxymethyl, methoxyethyl, ethoxyethyl, propoxyethyl, methoxypropyl, ethoxypropyl, propoxypropyl, methoxybutyl or ethoxybutyl), a di-$C_{1-4}$ alkoxy-methyl group (e.g. dimethoxymethyl, diethoxymethyl, dipropoxymethyl or dibutoxymethyl), a $C_{1-4}$ alkyl-carbamoyloxy-$C_{1-4}$ alkyl group (e.g. methylcarbamoyloxymethyl, ethylcarbamoyloxymethyl, propylcarbamoyloxymethyl, butylcarbamoyloxymethyl, methylcarbamoyloxyethyl, ethylcarbamoyloxyethyl, methylcarbamoyloxypropyl, ethylcarbamoyloxypropyl, methylcarbamoyloxybutyl or ethylcarbamoyloxybutyl), a $C_{1-4}$ alkyl-thiocarbamoyloxy-$C_{1-4}$ alkyl group (e.g. methylthiocarbamoyloxymethyl, ethylthiocarbamoyloxymethyl, propylthiocarbamoyloxymethyl, butylthiocarbamoyloxymethyl, methylthiocarbamoyloxyethyl, ethylthiocarbamoyloxyethyl, methylthiocarbamoyloxypropyl, ethylthiocarbamoyloxypropyl, methylthiocarbamoyloxybutyl or ethylthiocarbamoyloxybutyl), a formyl group, a hydroxyiminomethyl group or a cyano group; and

Each of X, Y and Z is a member of the ring A, represents N or CH and forms tetrazolo-, triazolo-, imidazo- or pyrazolo-ring.

The ring in the formula (I) and in the specification of the present invention represented by the formula:

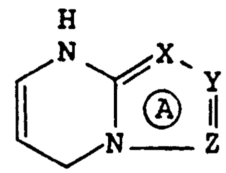

means a 4,7-dihydrotetrazolo[1,5-a]pyrimidine of the formula (a):

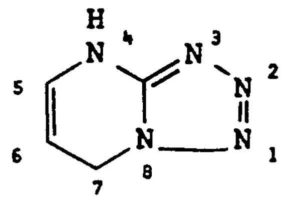

a 4,7-dihydrotriazolo[1,5-a]pyrimidine of the formula (b):

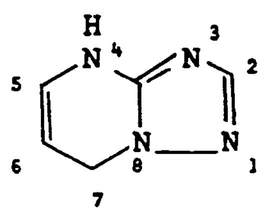

a 5,8-dihydroimidazo[1,2-a]pyrimidine of the formula ():

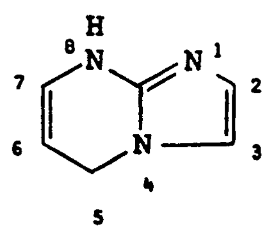

a 1,4-dihydroimidazo[1,5-pyrimidine of the formula (d):

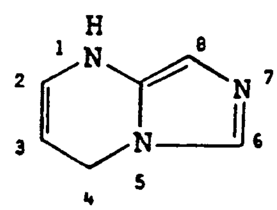

or a 4,7-dihydropyrazolo[1,5-a]pyrimidine of the formula (e):

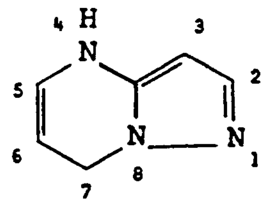

The compounds of formula (I) of the present invention can be, for example, prepared by the following methods.

(1) A method which comprises subjecting a compound of the formula:

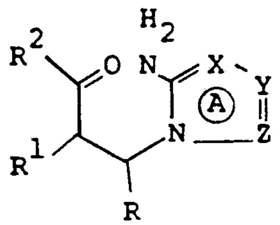

(II)

to dehydration condensation at room temperature or under heating in a suitable solvent or without it.

(2) A method which comprises subjecting a compound of the formula:

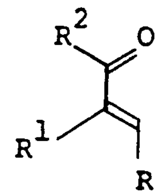

(III)

to condensation with a compound of the formula:

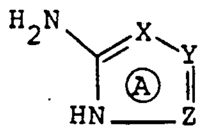

(IV)

at room temperature or under heating in a suitable solvent or without it.

(3) A method which comprises subjecting a compound of the formula:

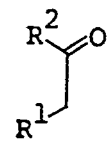

(V)

to dehydration condensation with a compound of the formula:

R—CHO (VI)

at room temperature or under heating in a suitable solvent or without it, if necessary in the presence of a suitable acid ctalyst, a base catalyst or both, and then subjecting the compound obtained to condensation with a compound of the formula (IV) at room temperature or under heating in a suitable solvent or without it.

(4) A method which comprises reacting a compound of the formula:

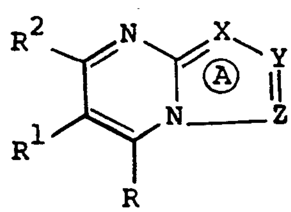

(VII)

with hydrogen gas in the presence of a suitable metal catalyst or a complex metal hydride (e.g. lithium aluminum hydride or sodium borohydride) at room temperature or under heating in a suitable solvent or without it.

(5) A method which comprises converting the substituents R, $R^1$ and $R^2$ of the compound obtained by the above-mentioned methods (1)—(4) according to usual manners of organic chemical synthesis.

Any invert solvent can be employed as a suitable solvent in the methods (1)—(4), and includes, for example, a lower alkanol (e.g. methanol, ethanol, propanol, isopropanol, butanol, tert-butanol or ethylene glycol), an aromatic hydrocarbon (e.g. benzene, toluene or xylene), an ether (e.g. diethyl ether, diisopropyl ether, dimethoxyethane, diethylene glycol dimethyl ether, tetrahydrofuran or dioxane), a halogenated lower alkane (e.g. dichloromethane, dichloroethane, chloroform or tetrachloromethane), an acid amide (e.g. dimethylformamide, N-methylpyrrolidone or hexamethylphosphoro triamide), a lower

alkanecarboxylic acid or an ester thereof (e.g. formic acid, acetic acid, propionic acid, methyl acetate, ethyl acetate, butyl acetate or methyl propionate), a nitrated lower alkane (e.g. nitromethane or nitroethane), dimethylsulfoxide, sulforane, water, or a mixture thereof.

The reactions of the methods (1)—(4) perform at a temperature under 200°C, and preferably at a temperature of from room temperature to 150°C.

The usual manners of organic chemical synthesis in the method (5) include, for example, formation of an aldehyde, a ketone, a carboxylic acid or an alcohol by hydrolysis of an acetal, a ketal or an ester in the presence of an acid or a base catalyst; formation of an aldehyde, a ketone, a carboxylic acid or an alcohol by removal of the protective group from the protected aldehyde, ketone, carboxylic acid or alcohol; formation of an oxime from an aldehyde or a ketone; reduction of an aldehyde or a nitro to an alcohol or an amine; formation of a nitrile by dehydration of an oximde or an amide; halogenation of an alkyl; acetoxylation of a haloalkyl; and formation of an urethane from an alcohol and an isocyanate.

The compounds of formula (I) having an asymmetric carbon atom can be prepared as a racemate or an optically active isomer. The racemate can be divided into a desired optical isomer by means of a fractional recrystallization of a salt with an optically active acid. The compounds of formula (I) having at least two asymmetric carbon atoms can be prepared as an individual diastereomer or a mixture thereof. The individual diastereomer can be purified by means of fractional recrystallization or chromatography. In the compound (I) having an oxime of the present invention, syn- and anti-stereoisomers can exist. The compounds of the present invention also include the syn- and anti-forms.

The compounds of formula (I), optical isomers thereof or diastereomers thereof can, if desired, be converted into acid addition salts thereof with an inorganic acid (e.g. hydrochloric aid, hydrobromic acid, sulfuric acid or phosphoric acid), an organic acid (e.g. acetic acid, propionic acid, glycollic acid, succinic acid, maleic acid, fumaric acid, malic acid, tartaric acid, oxalic acid, citric acid, benzoic acid, cinnamic acid, mandelic aid, salicylic acid, 2-acetoxybenzoic acid, nicotinic acid or isonicotinic acid), an organic sulfonic acid (e.g. methanesulfonic acid, ethanesulfonic acid, 2-hydroxyethanesulfonic acid, benzenesulfonic acid, p-toluenesulfonic acid or naphthalene-2-sulfonic acid), ascorbic acid, lysine or glutamic acid. Furthermore, the compounds (I) wherein $R^1$ is a carboxyl group can also be converted into metal salts thereof (e.g. sodium salt, potassium salt or aluminum salt) or salts with amines (e.g. a salt with triethylamine).

Since the compounds of the present invention exhibit antihypertensive, cardiovasodilative, cerebrovasodilative, cardiotonic and lipid peroxidation inhibiting activity as shown in the following pharmacological experiments, they are useful as drugs for the prophylaxis and the treatment of circulatory diseases such as antihypertensive agents, cardiotonics, drugs for the treatment of angina pectoris, heat failure or cardiac infarction, cerebral circulation improvers, prophylactics for the blood vessel, and so on.

The following pharmacological experiments explain the effects of the compounds according to the present invention in more detail. The test compounds employed are as follows:

Compound A: 1-Phenyl-2-piperidinoethyl 7-methyl-5-(3-nitrophenyl)-5,8-dihydroimidazo[1,2-a]pyrimidine-6-carboxylate (β-form of diastereomer)

Compound B: 1-Phenyl-2-piperidinoethyl 5-methyl-7-(3-nitrophenyl)-4,7-dihydropyrazolo[1,5-a]pyrimidine-6-carboxylate (β-form of diastereomer)

Compound C: 6-Ethoxycarbonyl-5-methyl-7-(2-trifluoromethylphenyl)-4,7-dihydropyrazolo[1,5-a]pyrimidine

Compound D: 6-Ethoxycarbonyl-5,7-dimethyl-5,8-dihydroimidazo[1,2-a]pyrimidine

Pharmacological experiment 1: Effect on hypertension

Each of 30 mg/kg of test compound B was orally administered to five spontaneously hypertensive rats and the blood pressures were measured by tail cuff method. The mean blood pressure lowering value after 1 hour administration was 94 mmHg and that value after 5 hours administration was 47 mmHg.

Pharmacological experiment 2: Effect on coronary blood flow

Adult mongrel dogs were anesthetized with sodium pentobarbital (30 mg/kg, i.v.). According to the method of Yago et al described in Folia Pharmacol. Japon, vol 57, 580 (1961), the left coronary artery was perfused and its blood flow was measured. Test compound was injected into the coronary artery at a volume of 10—31 μl.

The effects of test compound on coronary blood flow were expressed as $ED_{50}$ (μg), a dose required to increase the coronary blood flow by a half of the effects of 3 μg of nifedipine (dimethyl 2,6-dimethyl-4-(2-nitrophenyl)-1,4-dihydropyridine-3,5-dicarboxylate). Each of half-life (T 1/2, minute) was measured as the duration of effects. The results are summarized in Table 1.

Table 1

| Test Compound | $ED_{50}$ (μg, in artery) | T 1/2 (min.) |
|---|---|---|
| A | 10 | 5 |
| B | 1.7 | 1.5 |

Pharmacological experiment 3: Effect on vertebral blood flow

Adult mongrel dogs were anesthetized with sodium pentobarbital (25 mg/kg, i.v.). The right vertebral artery was perfused and the blood flow was measured. Test compound was injected in the vertebral artery. The percentage of maximum increase of blood flow by the injection of 100 μg of papaverine hydrochloride (1-[(3,4-dimethoxyphenyl)methyl]-6,7-dimethoxyisoquinoline hydrochloride) in vertebral artery was taken as 100%. The effects of test compound on vertebral artery were expressed as $ED_{100}$ (μg), a dose required to obtain 100% of the increase of blood flow. Each of half-life (T 1/2, minute) was measured as the duration of the effects. The results are summarized in the following Table 2.

Table 2

| Test Compound | $ED_{100}$ (μg, in artery) | T 1/2 (min.) |
|---|---|---|
| A | 3.5 | 0.9 |
| B | 1.0 | 3.0 |

Pharmacological experiment 4: Effect on myocardial contractile force

The papillary muscle of the rabbit right ventricle was isolated and suspended in Krebs-Henseleit solution (consisting of 117 mM sodium chloride, 4.7 mM potassium chloride, 2.6 mM calcium chloride, 1.4 mM magnesium sulfate, 1.2 mM potassium dihydrogenphosphate, 24.9 mM sodium hydrogencarbonate and 11 mM glucose). The solution was maintained at 35°C and equilibrated with oxygen gas containing 5% of carbonic acid gas, and then the preparations were stimulated at frequency of 0.5 Hz. The developed tensions were measured with a force development transducer under a preload of 0.5 g. The results are summarized in the Table 3.

Table 3

| Test Compound | Concentration (M) | Number of animals | Increase in contractile force (%) |
|---|---|---|---|
| C | $10^{-5}$ | 4 | 54.2 |
| | $10^{-4}$ | 4 | 132.5 |
| D | $10^{-5}$ | 3 | 21.7 |
| | $10^{-4}$ | 3 | 86.0 |

Pharmacological experiment 5: Effect on heart rate

Adult mongrel dogs were anesthetized with sodium pentobarbital (i.v.). Heart rate was measured from electrocardiogram (lead II). Test compound was injected into the right coronary artery. The results are summarized in the Table 4.

Table 4

| Test Compound | Dose (μg) | Increase in heart rate (%) |
|---|---|---|
| C | 30 | -7.1 |
| | 100 | -10.5 |

Pharmacological experiment 6: Effect on lipid peroxidation

Male Sprague-Dawley rats weighing 300—350 g were used. According to the method of Shimada et al described in Biochim. Biophys. Acta, vol. 572, 531 (1979), the liver mitochondria were isolated and ascorbic acid, ferrous sulfate, potassium chloride and Tris-HCl buffer were added. To the solution was added test compound dissolved in dimethylsulfoxide, and the mixture was incubated at 25°C for 30 minutes. The amount of malondialdehyde was determined by the thiobarbituric method.

The 50% inhibitory concentration ($IC_{50}$, μmol) of test compound on the formation of malondialdehyde was 6 μmol.

Experiment of acute toxicity:

Test compound C was orally or intraperitoneally administered to ddY strain mice. All mice survived at the oral dose of 1,000 mg/kg and at the intraperitoneal dose of 250 mg/kg for five days after administration.

The compounds of the present invention, when used as drugs, can be administered in the form of powder, tablets, capsules, granules, injectable solutions, suppositories, ointments or the like by mixing with a suitable carrier, excipient, diluent or the like. The dose for human adults usually ranges from 10 mg to 500 mg, but it may vary depending upon the age, body weight, and severity of the conditions to be treated.

Formulation Example 1: Tablets

| | 25 mg tablets | 50 mg tablets |
|---|---|---|
| Compound (I) | 25.0 mg | 0 50.0 mg |
| Lactose | 59.5 | 67.0 |
| Corn starch | 20.0 | 25.0 |
| Crystalline cellulose | 10.0 | 20.0 |
| Methyl cellulose | 1.0 | 1.5 |
| Talc | 4.0 | 6.0 |
| Magnesium stearate | 0.5 | 0.5 |
| | 120.0 mg | 170.0 mg |

Formulation Example 2: 10% Powder

| | |
|---|---|
| Compound (I) | 10.0% |
| Lactose | 59.5 |
| Corn starch | 30.0 |
| Talc | 0.5 |
| | 100.0% |

The present invention will be explained by the following examples in more detail, but these examples ae not to be construed as limiting the present invention:

Example 1

To 1-phenyl-2-piperidinoethyl α-acetyl-3-nitrocinnamate prepared from 7.5 g of 3-nitrobenzaldehyde and 14.5 g of 1-phenyl-2-piperidinoethyl acetoacetate is added a solution of 4.2 g of 3-aminopyrazole in ethanol, and then stirred at 50°C for 3 hours. The mixture is concentrated at reduced pressure, and the residue is purified by column chromatography on silica gel with chloroform-ethyl acetate-ethanol eluents to give two forms of diastereomers. The initially eluated solution is concentrated and the residue is recrystallized from ethanol to give one form (α-form) of diastereomer of 1-phenyl-2-piperidinoethyl 5-methyl-7-(3-nitrophenyl)-4,7-dihydropyrazolo[1,5-a]pyrimidine-6-carboxylate, melting at 92—93°C. Then, the subsequently eluated solution is concentrated and the residue is recrystallized from ethanol to give the other form (β-form) of diastereomer of 1-phenyl-2-piperidinoethyl 5-methyl-7-(3-nitrophenyl)-4,7-dihydropyrazolo[1,5-a]pyrimidine-6-carboxylate, melting at 191—192°C.

Example 2

Ethyl α-acetyl-β-(3-aminopyrazol-2-ly)-β-(2-trifluoromethylphenyl)propionate (34.5 g) is dissolved in 200 ml of chloroform and heated under reflux for 6 hours. After the reaction mixture is washed with water, dried and concentrated, the residue is purified by column chromatography on silica gel with chloroform-methanol eluents to give 24 g of the product. The product is crystallized from isopropylether and further recrystallized from ethanol to give 6-ethoxycarbonyl-5-methyl-7-(2-trifluoromethylphenyl)-4,7-dihydropyrazolo[1,5-a]pyrimidine, melting at 182—183°C.

Ethyl α-acetyl-β-(3-aminopyrazol-2-ly)-β-(2-trifluoromethylphenyl)propionate is synthesized by the following manner.

Ethyl α-acetyl-2-trifluoromethylcinnamate (36 g) and 10.5 g of 3-aminopyrazole are dissolved in 200 ml of ethanol, and the mixture is stirred at room temperature for 5 hours. The precipitated white crystals are collected by filtration to give 8.5 g of ethyl α-acetyl-β-(3-aminopyrazol-2-ly)-β-(2-trifluoromethylphenyl)-propionate, melting at 136—137°C.

Example 3

To the crude 1-phenyl-2-piperidinoethyl α-acetyl-3-nitrocinnamate, which was synthesized by reacting 30 g of 3-nitro-benzaldehyde with 67 g of 1-phenyl-2-piperidinoethyl acetoacetate, is added 500 ml of ethanol solution of 2-aminoimidazole prepared from 26.4 g of 2-aminoimidazole sulfate and sodium hydroxide, and then stirred at 50°C for 5 hours. After cooling, the precipitated crystals are collected by filtration and recrystallized from ethanol to give 9.7 g of 1-phenyl-2-piperidinoethyl 7-methyl-5-(3-nitrophenyl)-5,8-dihydroimidazo[1,2-a]pyrimidine-6-carboxylate, melting at 205—206°C as one diastereomer (α-form). Then, the filtrate is concentrated under reduced pressure, and the residue is purified by column chromatography on silica gel with chloroform-ethyl acetate-ethanol eluents. The crystals thus obtained are recrystallized from a mixture of chloroform and ethanol to give 4.8 g of 1-phenyl-2-piperidinoethyl 7-methyl-5-(3-nitrophenyl)-5,8-dihydroimidazo[1,2-a]pyrimidine-6-carboxylate, melting at 216—217°C with decomposition as the other diastereomer (β-form).

Example 4

To a solution of 10.5 g of 2-aminoimidazole sulfate in 11 ml of water is added a sodium ethoxide solution prepared from 1.7 g of metal sodium and 200 ml of ethanol. After the precipitated crystals are filtered off, to the filtrate is added 15.5 g of ethyl α-acetylcrotonate and then stirred at 50°C for 3 hours and at 75°C for 3 hours. After the ethanol is evaporated at reduced pressure, the residue is extracted by chloroform, washed with water and dried. The solvent is distilled off under reduced pressure to give white crystals. The crystals are recrystallized from ethanol to give 10.2 g of 6-ethoxycarbonyl-5,7-dimethyl-5,8-dihydroimidazo[1,2-a]pyrimidine, melting at 185—186.5°C.

Example 5

To 50 ml of ethanol are added 2.6 g of ethyl 3-nitro-α-acetylcinnamate and 0.9 g of 5-aminotetrazole, and then heated under reflux for 16 hours. On cooling, 2 g of 6-ethoxycarbonyl-5-methyl-7-(3-nitrophenyl)-4,7-dihydrotetrazolo[1,5-a]pyrimidine, melting at 210—212°C, is obtained.

Example 6

To 50 ml of ethanol are added 7.8 g of ethyl α-acetylcrotonate and 4.2 g of 3-amino-1,2,4-triazole, stirred at 50°C for an hour and then heated under reflux for an hour. The ethanol is evaporated under reduced pressure, and the obtained crystals are recrystallized from ethanol to give 7 g of 6-ethoxycarbonyl-5,7-dimethyl-4,7-dihydro-1,2,4-triazolo[1,5-a]pyrimidine, melting at 155—157°C.

Example 7

A solution of 6.7 g of 6-ethoxycarbonyl-5,7-dimethyl-4,7-dihyydro-1,2,4-triazolo[1,5-a]pyrimidine in 40 ml of acetic acid is heated at 50°C. To the reaction mixture is added dropwise a solution of 4.8 g of bromine

in 20 ml of acetic acid for 10 minutes, and the mixture is stirred at 50—60°C for an hour. The resulting mixture is poured into water, extracted with chloroform, washed with water and dried. The chloroform layer is purified by column chromatography on silica gel to give 0.5 g of 5-dibromomethyl-6-ethoxycarbonyl-7-methyl-4,7-dihydro-1,2,4-triazolo[1,5-a]pyrimidine, melting at 170—171°C with decomposition.

Example 8

A mixture of 50 g of methyl 2-acetyl-3-(2-thienyl)acrylate, 20 g of 3-amino-1,2,4-triazole and 300 ml of ethanol is heated under reflux for 4 hours. The precipitated white crystals are collected by filtration to give 29.5 g of 6-methoxycarbonyl-5-methyl-7-(2-thienyl)-4,7-dihydro-1,2,4-triazolo[1,5-a]pyrimidine, melting at 239—240°C.

Methyl 2-acetyl-3-(2-thienyl)acrylate was synthesized by the following manner.

A mixture of 112 g of 2-thiophenecarbaldehyde, 116 g of methyl acetoacetate, 770 ml of benzene, 4 ml of acetic acid and 3 ml of piperidine is heated under reflux for 2.5 hours under the condition of azeotropic dehydration. The benzene is evaporated under reduced pressure and the residual solvent is distilled off to give 199 g of methyl 2-acetyl-3-(2-thienyl)acrylate, boiling at 130—160°C/0.2 mmHg.

Example 9

In 300 ml of acetic acid is dissolved 29.5 g of 6-methoxycarbonyl-5-methyl-7-(2-thienyl)-4,7-dihydro-1,2,4-triazolo[1,5-a]pyrimidine at 60°C. To the solution is added dropwise a solution of 16 g of bromine in 50 ml of acetic acid for 20 minutes with stirring. After stirring at room temperature for an hour, the reaction mixture is poured into water, extracted with chloroform, washed with water and potassium carbonate solution, dried and the solvent is evaporated under reduced pressure. The obtained pale yellow crystals are recrystallized from ethanol to give 18 g of 5-bromomethyl-6-methoxycarbonyl-7-(2-thienyl)-4,7-dihydro-1,2,4-triazolo[1,5-a]pyrimidine, melting at 155°C with decomposition.

Example 10

A mixture of 29.2 g of benzalacetone and 17.6 g of 3-amino-1,2,4-triazole is refluxed in 150 ml of dimethylformamide for 3 hours. On cooling at room temperature, crystals are precipitated and collected by filtration. The crystals are recrystallized from dimethylformamide to give 17 g of 5-methyl-7-phenyl-4,7-dihydro-1,2,4-triazole[1,5-a]pyrimidine, melting at 220—222°C.

Example 11

To a solution of 41.3 g of 2-aminoimidazole sulfate in 50 ml of water are added 6.8 g of sodium hydroxide and 500 ml of methanol, and the mixture is stirred and dried over sodium sulfate. To the resulting solution is added 77.9 g of methyl α-acetyl-3-nitrocinnamate, stirred at 50°C for 2.5 hours and refluxed under heating for 1.5 hours. On cooling at room temperature, crystals are precipitated and collected by filtration to give 81.4 g of 6-methoxycarbonyl-7-methyl-5-(3-nitrophenyl)-5,8-dihydroimidazo[1,2-a]pyrimidine, melting at 263—264°C with decomposition.

Example 12

A mixture of 20.0 g of 6-methoxycarbonyl-7-methyl-5-(3-nitrophenyl)-5,8-dihydroimidazo[1,2-a]-pyrimidine, 200 ml of acetic acid and 3.0 g of 10 palladium-carbon is stirred at room temperature for 5 hours under hydrogen gas. After an insoluble material is filtered off, the filtrate is neutralized with sodium bicarbonate, and extracted with chloroform. The extract is dried and the solvent is distilled off under reduced pressure. The precipitated pale yellow crystals are collected by filtration and recrystallized from methanol to give 11.0 g of 5-(3-aminophenyl)-6-methoxycarbonyl-7-methyl-5,8-dihydroimidazo[1,2-a]-pyrimidine, melting at 224—225°C.

Example 13

To a solution of 4.0 g of 2-aminoimidazole sulfate in 10 ml of water are added 1.2 g of sodium hydroxide and 200 ml of methanol, and the mixture is dried over sodium sulfate. To the resulting solution is added 9.5 g of 2-methoxyethyl α-acetyl-2-trifluoromethylcinnamate and stirred at 50°C for 3 hours. After stirring under reflux for 3.5 hours, the solvent is distilled off and the precipitated crystals are collected by filtration. The crystals are recrystallized from a mixture of methanol and chloroform to give 5.5 g of 6-(2-methoxyethoxycarbonyl)-7-methyl-5-(2-trifluoromethylphenyl)-5,8-dihydroimidazo[1,2-a]pyrimidine, melting at 181—182°C.

2-Methoxyethyl α-acetyl-2-trifluoromethylcinnamate is synthesized by the following manner.

A mixture of 12.2 g of 2-trifluromethylbenzaldehyde, 11.2 g of 2-methoxyethyl acetoacetate, 1 ml of piperidine, 2 ml of acetic acid and 150 ml of benzene is refluxed under the condition of azeotropic dehydration for 3.5 hours. The solvent is distilled off under reduced pressure to give red brown oil. The oil is distilled off under reduced pressure to give 15.5 g of 2-methoxyethyl α-acetyl-2-trifluoromethyl-cinnamate, boling at 145—151°C/0.5 mmHg.

Example 14

Crystals are obtained by reacting 6.8 g of 2-aminoimidazole sulfate with 10.5 g of methyl 2-acetyl-3-cyclohexylacrylate in a similar manner as described in Example 13. Recrystallization from methanol gives 4.8 g of 5-cyclohexyl-6-methoxycarbonyl-7-methyl-5,8-dihydroimidazo[1,2-a]pyrimidine, melting at 178°C.

Example 15

To 100 ml of benzene are added 10.7 g of 2-pyridinecarbaldehyde and 13 g of methyl acetoacetate, and maintained at 30—40°C for 2 hours in the presence of 0.5 ml of acetic acid and 0.25 ml of piperidine. The mixture is washed with sodium chloride solution, dried and the solvent is distilled off. To the residue is added 150 ml of isopropanol solution containing 2-aminoimidazole prepared from 10.5 g of 2-aminoimidazole sulfate and 2.9 g of sodium hydroxide, and then refluxed under heating for 2 hours. The reaction mixture is passed through silica gel column, and the solvent of the eluate is distilled off. Crystallization from methanol gives 2.5 g of 6-methoxycarbonyl-7-methyl-5-(2-pyridyl)-5,8-dihydroimidazo[1,2-a]pyrimidine, melting at 224—226°C with decomposition.

Example 16

2-N,N-diethylaminoethyl 5-(3-nitrophenyl)-7-methyl-5,8-dihydroimidazo[1,2-a]pyrimidine-6-carboxylate (1.6 g), melting at 119—121°C is obtained as crystals by reacting 7.6 g of 3-nitrobenzaldehyde with 10.1 g of 2-N,N-diethylaminoethyl acetoacetate and 4.5 g of 2-aminoimidazole sulfate in a similar manner as described in Example 15.

Example 17

In a mixture of 50 ml of acetone, 30 ml of methanol and 20 ml of water is dissolved 1.8 g of 5 - [3 - (2 - methyl - 1,3 - dioxolan - 2 - yl)phenyl] - 6 - methoxycarbonyl - 7 - methyl - 5,8 - dihydroimidazo[1,2 - a]pyrimidine. The mixture is adjusted to pH 1 with p-toluenesulfonic acid and then heated at 60°C for 3 hours. The solvent is distilled off and the residue is neutralized with sodium bicarbonate solution. The resulting white precipitations are collected by filtration and recrystallized from a mixture of ethanol and acetone to give 1.1 g of 5 - (3 - acetylphenyl) - 6 - methoxycarbonyl - 7 - methyl - 5,8 - dihydroimidazo[1,2 - a]pyrimidine, melting at 227—229°C.

5 - [3 - (2 - methyl - 1,3 - dioxolan - 2 - yl)phenyl] - 6 - methoxycarbonyl - 7 - methyl - 5,8 - dihydroimidazo[1,2 - a]pyrimidine is synthesized according to the following manner.

5 - [3 - (2 - methyl - 1,3 - dioxolan - 2 - yl)phenyl] - 6 - methoxycarbonyl - 7 - methyl - 5,8 - dihydroimidazo[1,2 - a]pyrimidine (9 g), melting at 199—201°C, is obtained by reacting 11 g of 3-(2-methyl-1,3-dioxolan-2-yl)benzaldehyde with 7.3 g of methyl acetoacetate and 9 g of 2-aminoimidazole sulfate in a similar manner as described in Example 15.

Example 18

To an isopropanol solution of 2-aminoimidazole, which is prepared from 7.9 g of 2-aminoimidazole sulfate and 2.2 g sodium hydroxide, is added 11.7 g of α-acetyl-3-nitrocrotonamide and heated under reflux for 5 hours. After cooling, the precipitated crystals are collected by filtration to give 12 g of 6-carbamoyl-7-methyl-5-(3-nitrophenyl)-5,8-dihydromidazo[1,2-a]pyrimidine, melting at 238—240°C.

α-Acetyl-3-nitrocrotonamide is synthesized by the following manner.

A mixture of 10 g of acetoacetamide and 15 g of 3-nitrobenzaldehyde in 100 ml of isopropanol is stirred at 35°C for 4 hours in the presence of 0.3 ml of piperidine and 0.5 ml of acetic acid. The precipitated yellow crystals are collected by filtration to give 20 g of α-acetyl-3-nitrocrotonamide, melting at 159—161°C.

Example 19

1.5 ml of thionyl chloride is added dropwise to a solution of 3 g of 6-carbamoyl-7-methyl-5-(3-nitrophenyl)-5,8-dihydroimidazo[1,2-a]pyrimidine in 30 ml of dimethylformamide. After stirring for an hour, the mixture is poured into chloroform and washed with sodium bicarbonate solution followed by passing the mixture through a column containing 50 g of silica gel with a mixture of dichloromethane and ethanol as an eluent. Upon allowing the eluates to stand, crystals are precipitated and collected by filtration to give 1.1 g of 6-cyano-7-methyl-5-(3-nitrophenyl)-5,8-dihydroimidazo[1,2-a]pyrimidine, melting at 278—280°C with decomposition.

Example 20

50 ml of ethanol is added to a solution of 4.5 g of 2-aminoimidazole sulfate and 1.3 g of sodium hydroxide in 11 ml of water and dried over sodium sulfate. To the solution is added 10 g of ethyl 4,4-dimethyl-2-(3-nitrobenzylidene)-3-oxobutylate. The resulting mixture is stirred at 50°C for 30 minutes and further refluxed under heating for 6 hours. After cooling, the precipitated yellow crystals are collected by filtration, purified by column chromatography on silica gel with chloroform-methanol eluents, and then recrystallized from methanol to give 6 g of 7-dimethoxymethyl-6-ethoxycarbonyl-5-(3-nitrophenyl)-5,8-dihydroimidazo[1,2-a]pyrimidine, melting at 190—191°C with decomposition.

Ethyl 4,4-dimethoxy-2-(3-nitrobenzylidene)-3-oxobutylate is synthesized by the following manner.

A solution of 24 g of 3-nitrobenzaldehyde, 30 g of ethyl 4,4-dimethoxy-3-oxobutylate, 0.5 ml of piperidine and 1 ml of acetic acid in 200 ml of benzene is refluxed under heating for 4 hours under the condition of azeotropic dehydration. The solvent is distilled off and evaporated under reduced pressure to give 36 g of ethyl 4,4-dimethoxy-2-(3-nitrobenzylidene)-3-oxobutylate, boiling at 195—210°C/0.2 mmHg.

Example 21

10 ml of 6N hydrochloric acid is added to a soltuion of 4.4 g of 7-dimethoxymethyl-6-ethoxycarbonyl-5-(3-nitrophenyl)-5,8-dihydroimidazo[1,2-a]pyrimidine in mixture of 10 ml of acetone and 10 ml of water and the mixture is stirred at room temperature for an hour, and then allowed to stand overnight. The solvent is distilled under reduced pressure below 40°C to give 4 g of 6-ethoxycarbonyl-7-formyl-5-(3-nitrophenyl)-5,8-dihydroimidazo[1,2-a]pyrimidine hydrochloride, melting at 228°C with decomposition.

Example 22

To a solution of 6-ethoxycarbonyl-7-formyl-5-(3-nitrophenyl)-5,8-dihydroimidazo[1,2-a]pyrimidine hydrochloride in a mixture of 20 ml of ethanol and 20 m of dimethylformamide are added 0.88 g of hydroxylamine hydrochloride and 1.22 g of sodium carbonate and the mixture is stirred at 50°C for 1.5 hours. The solvent is distilled off under reduced pressure and water is added to the residue, then precipitated crystals are collected by filtration. The crystals are recrystallized from a mixture of chloroform and methanol to give 2.0 g of 6-ethoxycarbonyl-7-hydroxyiminomethyl-5-(3-nitrophenyl)-5,8-dihydroimidazo[1,2-a]pyrimidine, melting at 229—230°C with decomposition.

Example 23

A solution of 6-ethoxycarbonyl-7-hydroxyiminomethyl-5-(3-nitrophenyl)-5,8-dihydroimidazo[1,2-a]pyrimidine (1.25 g), 0.29 g of sodium acetate and 0.4 ml of acetic anhydride in 15 ml of acetic acid is stirred with heating at 80—90°C for 30 minutes. The reaction solution is poured into water and precipitated crystals are collected by filtration. Recrystallization from a mixture of chloroform and ethanol gives 0.5 g of 7-cyano-6-ethoxycarbonyl-5-(3-nitrophenyl)-5,8-dihydroimidazo[1,2-a]pyrimidine, melting at 260—261°C with decomposition.

Example 24

12 g of 8-carboxy-3-ethoxycarbonyl-2-methyl-4-(3-nitrophenyl)-1,4-dihydroimidazo[1,5-a]pyrimidine is heated at 215°C for 20 minutes and purified by column chromatography on silica gel with chloroform eluent. The yellow crystals obtained are recrystallized from ethanol to give 1.5 g of 3-ethoxycarbonyl-2-methyl-4-(3-nitrophenyl)-1,4-dihydroimidazo[1,5-a]pyrimidine, melting at 196—197°C.

8-Carboxy-3-ethoxycarbonyl-2-methyl-4-(3-nitrophenyl)-1,4-dihydroimidazo[1,5-a]pyrimidine is synthesized by the following manner.

0.17 g of potassium hydroxide in 1.7 ml of water is added to a solution of 1.0 g of 3,8-diethoxycarbonyl-2-methyl-4-(3-nitrophenyl)-1,4-dihydroimidazo[1,5-a]pyrimidine in 20 ml of ethanol, and gradually heated followed by stirring at 70°C for 10 hours. The ethanol is distilled off under reduced pressure, chloroform is added to the resulting oil and extracted with potassium carbonate solution. The water layer is washed with chloroform and adjusted to pH 5—6 with acetic acid. The precipitated crystals are collected by filtration and recrystallized from ethanol to give 0.32 g of 8-carboxy-3-ethoxycarbonyl-2-methyl-4-(3-nitrophenyl)-1,4-dihydroimidazo[1,5-a]pyrimidine, melting at 222°C with decomposition.

3,8-Diethoxycarbonyl-2-methyl-4-(3-nitrophenyl)-1,4-dihydroimidazo[1,5-a]pyrimidine is synthesized by the following manner.

A solution of 2 g of 5-amino-4-ethoxycarbonylimidazole and 3.4 g of ethyl α-acetyl-3-nitrocinnamate in 50 ml of ethanol is refluxed under heating for 7 hours under a nitrogen atmosphere. The ethanol is removed under reduced pressure, the resulting oil is purified by column chromatography on silica gel with a mixture of chloroform and methanol as eluent. Recrystallization from a mixture of isopropyl alcohol and isopropyl ether gives 2.5 g of 3,8 - diethoxycarbonyl - 2 - methyl - 4 - (3 - nitrophenyl) - 1,4 - dihydroimidazo[1,5 - a]pyrimidine, melting at 160—162°C.

Example 25

16 mg of sodium borohydride is added gradually to a solution of 17 mg of 6-methoxycarbonyl-5-methyl-7-(2-trifluoromethylphenyl)pyrazolo[1,5-a]pyrimidine in 1 ml of methanol with stirring and heating at 60°C. The reaction mixture is poured into water, extracted with ethyl acetate and dried. The solvent is removed and the residue is recrystallized from methanol to give 6 - methoxycarbonyl - 5 - methyl - 7 - (2 - trifluoromethylphenyl) - 4,7 - dihydropyrazolo[1,5 - a]pyrimidine, melting at 209—210°C.

The following compounds can also be, for example, prepared in a similar manner as the above examples.

(26) 6-Ethoxycarbonyl-5-methyl-7-(3-nitrophenyl)-4,7-dihydropyrazolo[1,5-a]pyrimidine, melting at 157—159°C with decomposition

(27) 6-Ethoxycarbonyl-7-methyl-5-(3-nitrophenyl)-5,8-dihydroimidazo[1,2-a]pyrimidine, melting at 242—243°C

(28) 7-Chloromethyl-6-ethoxycarbonyl-5-(3-nitrophenyl)-5,8-dihydroimidazo[1,2-a]pyrimidine, melting at 190—192°C with decomposition

(29) 6-tert-Butoxycarbonyl-7-methyl-5-(3-nitrophenyl)-5,8-dihydroimidazo[1,2-a]pyrimidine, melting at 219—222°C with decomposition

(30) 6-(2-Cyanoethoxycarbonyl)-7-methyl-5-(3-nitrophenyl)-5,8-dihydroimidazo[1,2-a]pyrimidine, melting at 230—237°C

(31) 6-Ethoxycarbonyl-7-N-methylcarbamoyloxymethyl-5-(3-nitrophenyl)-5,8-dihydroimidazo[1,2-a]pyrimidine, melting at 207—208°C with decomposition

(32) 6-Ethoxycarbonyl-7-hydroxymethyl-5-(3-nitrophenyl)-5,8-dihydroimidazo[1,2-a]pyrimidine, melting at 196—197°C with decomposition

(33) 6-Ethoxycarbonyl-5-(3-nitrophenyl)-7-trifluoromethyl-5,8-dihydroimidazo[1,2-a]pyrimidine, melting at 240—242°C with decomposition

(34) 6-Benzyloxycarbonyl-7-methyl-5-(3-nitrophenyl)-5,8-dihydroimidazo[1,2-a]pyrimidine, melting at 205°C

(35) 6-(2-Methoxyethoxycarbonyl)-7-methyl-5-(3-nitrophenyl)-5,8-dihydroimidazo[1,2-a]pyrimidine, melting at 185—186°C

(36) 3-N,N-Dimethylaminopropyl 7-methyl-5-(3-nitrophenyl)-5,8-dihydroimidazo[1,2-a]pyrimidine-6-carboxylate

(37) 6-Ethoxycarbonyl-5-(3-nitrophenyl)-7-propyl-5,8-dihydroimidazo[1,2-a]pyrimidine

(38) 6-Carboxy-7-formyl-5-(3-nitrophenyl)-5,8-dihydroimidazo[1,2-a]pyrimidine hydrochloride, melting at above 350°C

(39) 7-Methyl-5-(3-nitrophenyl)-5,8-dihydroimidazo[1,2-a]pyrimidine, melting at 194—196°C with decomposition

(40) 6-Ethoxycarbonyl-7-methyl-5-(2-nitrophenyl)-5,8-dihydroimidazo[1,2-a]pyrimidine, melting at 253—254°C with decomposition

(41) 6-Methoxycarbonyl-7-methyl-5-(2-nitrophenyl)-5,8-dihydroimidazo[1,2-a]pyrimidine, melting at 256—258°C with decomposition

(42) 6-Methoxycarbonyl-7-methyl-5-(4-nitrophenyl)-5,8-dihydroimidazo[1,2-a]pyrimidine, melting at 249°C with decomposition

(43) 6-Ethoxycarbonyl-7-methyl-5-(2-trifluoromethylphenyl)-5,8-dihydroimidazo[1,2-a]pyrimidine, melting at 276°C

(44) 6-Methoxycarbonyl-7-methyl-5-(2-trifluoromethylphenyl)-5,8-dihydroimidazo[1,2-a]pyrimidine, melting at 285—286°C with decomposition

(45) 7-Acetoxymethyl-6-ethoxycarbonyl-5-(2-trifluoromethylphenyl)-5,8-dihydroimidazo[1,2-a]pyrimidine, melting at 174—176°C

(46) 2-Piperidinoethyl 7-methyl-5-(2-trifluoromethylphenyl)-5,8-dihydroimdazo[1,2-a]pyrimidine-6-carboxylate, melting at 205—207°C with decomposition

(47) 6-Ethoxycarbonyl-7-methyl-5-(3-trifluoromethylphenyl)5,8-dihydroimidazo[1,2-a]pyrimidine, melting at 193—194°C

(48) 7-Methyl-6-(2-nitroethoxycarbonyl)-5-(2-trifluoromethylphenyl)-5,8-dihydroimidazo[1,2-a]pyrimidine

(49) 5-(2-Benzylthiophenyl)-6-methoxycarbonyl-7-methyl-5,8-dihydroimidazo[1,2-a]pyrimidine, melting at 196—198°C

(50) 5-(2-Chlorophenyl)-6-methoxycarbonyl-7-methyl-5,8-dihydroimidazo[1,2-a]pyrimidine, melting at 257—258°C with decomposition

(51) 6-Ethoxycarbonyl-7-methyl-5-phenyl-5,8-dihydroimidazo[1,2-a]pyrimidine, melting at 183—184°C

(52) 6-Ethoxycarbonyl-7-methyl-5-(2-methylphenyl)-5,8-dihydroimidazo[1,2-a]pyrimidine, melting at 215°C

(53) 5-(2-Cyanophenyl)-6-methoxycarbonyl-7-methyl-5,8-dihydroimidazo[1,2-a]pyrimidine, melting at 268°C with decomposition

(54) 6-Methoxycarbonyl-7-methyl-5-(2-methylthiophenyl)-5,8-dihydroimidazo[1,2-a]pyrimidine, melting at 259—260°C with decomposition

(55) 5-(4-Aminophenyl)-6-methoxycarbonyl-7-methyl-5,8-dihydroimidazo[1,2-a]pyrmidine, pale yellow crystals

(56) 6-Methoxycarbonyl-5-(2-methoxyphenyl)-7-methyl-5,8-dihydroimidazo[1,2-a]pyrimidine, melting at 242—245°C with decomposition

(57) 6-Ethoxycarbonyl-7-methyl-5-propyl-5,8-dihydroimidazo[1,2-a]pyrimidine, melting at 144—145°C

(58) 6-Methoxycarbonyl-7-methyl-5-(2-thienyl)-5,8-dihydroimidazo[1,2-a]pyrimidine, melting at 230—232°C with decomposition

(59) 5-(2-Furyl)-6-methoxycarbonyl-7-methyl-5,8-dihydroimidazo[1,2-a]pyrimidine

(60) 6-Ethoxycarbonyl-7-methyl-5,8-dihydroimidazo[1,2-a]pyrimidine, melting at 248—249°C

(61) 7-Methyl-5-phenyl-5,8-dihydroimidazo[1,2-a]pyrimidine

(62) 6-Ethoxycarbonyl-5-methyl-7-(3-nitrophenyl)-4,7-dihydro-1,2,4-triazolo[1,5-a]pyrimidine, 222—224°C

(63) 5-Acetoxymethyl-6-ethoxycarbonyl-7-(3-nitrophenyl)-4,7-dihydro-1,2,4-triazolo[1,5-a]pyrimidine, melting at 169—171°C

(64) 6-Methoxycarbonyl-7-(3,4-dimethoxyphenyl)-5-methyl-4,7-dihydro-1,2,4-triazolo[1,5-a]pyrimidine, melting at 211—213°C

(65) 7-(2-Chlorophenyl)-6-methoxycarbonyl-5-methyl-4,7-dihydro-1,2,4-triazolo[1,5-a]pyrimidine, melting at 268—271°C with decomposition

(66) 6-Methoxycarbonyl-5-methyl-7-(2-trifluoromethylphenyl)-4,7-dihydro-1,2,4-triazolo[1,5a]pyrimidine, melting at 242°C

(67) 5-Bromomethyl-6-methoxycarbonyl-7-(2-trifluoromethylphenyl)-4,7-dihydro-1,2,4-triazolo[1,5-a]pyrimidine,

melting at 160—170°C

(68) 5-Chloromethyl-7-(4-hydroxyphenyl)-6-methoxycarbonyl-4,7-dihydro-1,2,4-trizolo[1,5-a]pyrimidine, melting at 290°C with decomposition

(69) 6-Ethoxycarbonyl-5-methyl-7-(3-pyridyl)-4,7-dihydro-1,2,4-triazolo[1,5-a]pyrimidine, melting at 221—223°C

(70) 5-tert-Butyl-7-(3-pyridyl)-4,7-dihydro-1,2,4-triazolo[1,5-a]pyrimidine, melting at 179—181°C

(71) 1-Phenyl-2-N,N-dibutylaminoethyl 7-methyl-5-(3-nitrophenyl)-5,8-dihydroimidazo[1,2-a]pyrimidine-6-carboxylate, α-form: melting at 146—147°C, β-form: melting at 174—175°C

(72) 1-(2-Thienyl)-2-piperidinoethyl 7-methyl-5-(3-nitrophenyl)-5,8-dihydroimidazo[1,2-a]pyrimidine-6-carboxylate, α-form: melting at 204—206°C, β-form: melting at 182—183°C

(73) 1-Phenyl-2-(N-methyl-N-benzylamino)ethyl 7-methyl-5-(3-nitrophenyl)-5,8-dihydroimidazo[1,2-a]pyrimidine-6-carboxylate, α-form: melting at 193—194°C, β-form: melting at 188—190°C

(74) 1-(2-Furyl)-2-piperidinoethyl 7-methyl-5-(3-nitrophenyl)-5,8-dihydroimidazo[1,2-a]pyrimidine-6-carboxylate, α-form: melting at 199—200°C with decomposition, β-form: melting at 186—187°C with decomposition

(75) 6-Carbamoyl-7-methyl-5-(2-trifluoromethylphenyl)-5,8-dihydroimidazo[1,2-a]pyrimidine, melting at 254—255°C

(76) 6-Cyano-7-methyl-5-(2-trifluoromethylphenyl)-5,8-dihydroimidazo[1,2-a]pyrimidine hydrochloride, melting at 275—278°C

(77) 6-Ethoxycarbonyl-5,7-dimethyl-4,7-dihydropyrazolo[1,5-a]pyrimidine, melting at 168—170°C

(78) 2-Methoxy-1-phenylethyl 7-methyl-5-(3-nitrophenyl)-5,8-dihydroimidazo[1,2-a]pyrimidine-6-carboxylate, α-form: melting at 194—195°C, β-form: melting at 184°C

(79) 6-Methoxycarbonyl-5-methyl-7-(3,4-dihydroxyphenyl)-4,7-dihydropyrazolo[1,5-a]pyrimidine, melting at 253—255°C

(80) 5-Acetoxymethyl-6-ethoxycarbonyl-7-(2-trifluoromethylphenyl)-4,7-dihydropyrazolo[1,5-a]pyrimidine, melting at 166—167°C

(81) 6-Ethoxycarbonyl-5-hydroxymethyl-7-(2-trifluoromethylphenyl)-4,7-dihydropyrazolo[1,5-a]pyrimidine, melting at 229—230°C

(82) 6-Methoxycarbonyl-5-methyl-7-(3-pyridyl)-4,7-dihydropyrazolo[1,5-a]pyrimidine, melting at 222—223°C

(83) 6-Ethoxycarbonyl-5-propyl-7-(2-thienyl)-4,7-dihydropyrazolo[1,5-a]pyrimidine, melting at 169—170°C

(84) 6-Ethoxycarbonyl-5-methyl-7-(2-chlorophenyl)-4,7-dihydropyrazolo[1,5-a]pyrimidine, melting at 176—178°C

(85) 6-Butoxycarbonyl-5-methyl-7-(2-trifluoromethylphenyl)-4,7-dihydropyrazolo[1,5-a]pyrimidine, melting at 134—136°C

(86) 6-Methoxycarbonyl-5-methyl-7-(2-hydroxyphenyl)-4,7-dihydropyrazolo[1,5-a]pyrimidine, melting at 218—220°C

(87) 5-Chloromethyl-6-ethoxycarbonyl-7-(2-trifluoromethylphenyl)-4,7-dihydropyrazolo[1,5-a]pyrimidine, melting at 147—149°C

(88) 6-Ethoxycarbonyl-5-fluoromethyl-7-(2-trifluoromethylphenyl)-4,7-dihydropyrazolo[1,5-a]pyrimidine

(89) 6-Acetyl-5-methyl-7-(3-nitrophenyl)-4,7-dihydropyrazolo[1,5-a]pyrimidine, melting at 190—192°C

(90) 6-Benzyloxycarbonyl-5-methyl-7-(2-trifluoromethylphenyl)-4,7-dihydropyrazolo[1,5-a]pyrimidine, melting at 164—166°C

(91) 6-Carboxy-5-methyl-7-(2-trifluoromethylphenyl)-4,7-dihydropyrazolo[1,5-a]pyrimidine, melting at 172—173°C with decomposition

(92) 6-Ethoxycarbonyl-5-methyl-7-(3-trifluoromethylphenyl)-4,7-dihydropyrazolo[1,5-a]pyrimidine, melting at 189—190°C

(93) 6-Ethoxycarbonyl-5-methyl-7-(2-methylphenyl)-4,7-dihydropyrazolo[1,5-a]pyrimidine, melting at 186—187°C

Although the present invention has been adequately discussed in the foregoing specification and examples included therein, one readily recognizes that various changes and modifications may be made without departing from the spirit and scope thereof.

**Claims**

1. A polyazaheterocycle derivative of the formula:

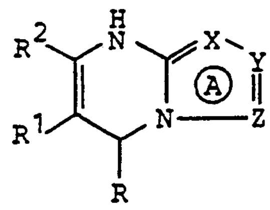

or a pharmaceutically acceptable salt thereof, wherein

R is a hydrogen atom, a $C_{1-5}$ alkyl group, a $C_{3-6}$ cycloalkyl group, a furyl group, a thienyl group, a pyridyl group or a phenyl group which may be optionally substituted by one or two substituents selected from the group consisting of a halogen atom, a $C_{1-4}$ alkyl group, a trifluoromethyl group, a $C_{1-4}$ alkoxy group, a phenyl-$C_{1-3}$ alkyl-oxy group, a $C_{1-4}$ alkyl-thio group, a phenyl-$C_{1-3}$ alkyl-thio group, a $C_{2-4}$ alkanoyl group, a benzoyl group, a hydroxyl group, an amino group, a nitro group and a cyano group;

$R^1$ is a hydrogen atom; a cyano group; a carbamoyl group; a carboxyl group; a $C_{1-4}$ alkoxy-carbonyl group which may be optionally substituted in the alkoxy moiety by one or two substituents selected from the group consisting of a $C_{1-4}$ alkoxy group, a cyano group, a group represented by the formula: —N($Ra^1$)($Rb^1$) (wherein each of $Ra^1$ and $Rb^1$ is a $C_{1-4}$ alkyl group or a phenyl-$C_{1-3}$ alkyl group, or $Ra^1$ and $Rb^1$ together with the adjacent nitrogen atom form a 5- to 7-membered heterocycle selected from the group consisting of pyrrolidine, piperidine, morpholine, piperazine, N-methylpiperazine, N-(2-hydroxyethyl)-piperazine, azepine and diazepine), a nitro group, a phenyl group and a heteroaryl group selected from the group consisting of thienyl, furyl and pyridyl; or a $C_{2-6}$ alkanoyl group selected from the group consisting of acetyl, propionyl, butyryl, isobutyryl, valeryl, isovaleryl, pivaloyl and hexanoyl;

$R^2$ is a $C_{1-5}$ alkyl group, a hydroxy-$C_{1-4}$ alkyl group, an acetoxy-$C_{1-4}$ alkyl group, a halo-$C_{1-4}$ alkyl group, a dihalo-$C_{1-4}$ alkyl group, a trifluoromethyl group, a $C_{1-4}$ alkoxy-$C_{1-4}$ alkyl group, a di-$C_{1-4}$ alkoxy-methyl group, a $C_{1-4}$ alkyl-carbamoyloxy-$C_{1-4}$ alkyl group, a $C_{1-4}$ alkylthiocarbamoyloxy-$C_{1-4}$ alkyl group, a formyl group, a hydroxyiminomethyl group or a cyano group; and

Each of X, Y and Z is a member of the ring A and represents N or CH.

2. The compound of claim 1 represented by the formula:

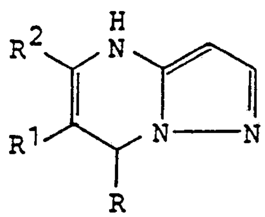

wherein each symbol is as defined in claim 1, or a pharmaceutically acceptable salt thereof.

3. The compound of claim 1 represented by the formula:

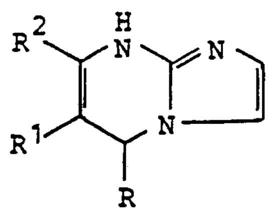

wherein each symbol is as defined in claim 1, or a pharmaceutically acceptable salt thereof.

4. The compound of claim 1 represented by the formula:

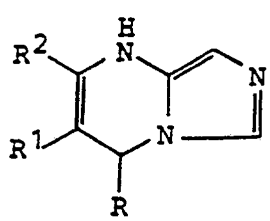

wherein each symbol is as defined in claim 1, or a pharmaceutically acceptable salt thereof.

5. The compound of claim 1: 1-phenyl-2-piperidinoethyl 5-methyl-7-(3-nitrophenyl)-4,7-dihydropyrazolo[1,5-a]pyrimidine-6-carboxylate and diastereomer thereof.

6. The compound of claim 1: 6-ethoxycarbonyl-5-methyl-7-(2-trifluoromethylphenyl)-4,7-dihydropyrazolo[1,5-a]pyrimidine.

7. The compound of the formula: 1-phenyl-2-piperidinoethyl 7-methyl-5-(3-nitrophenyl)-5,8-dihydroimidazo[1,2-a]pyrimidine-6-carboxylate and diastereomer thereof.

8. The compound of claim 1: 6-ethoxycarbonyl-5,7-dimethyl-5,8-dihydroimidazo[1,2-a]pyrimidine.

9. A method of preparing a polyazaheterocycle derivative of the formula:

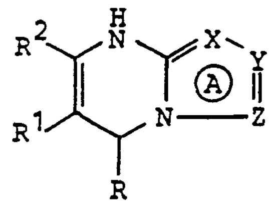

or a pharmaceutically acceptable salt thereof, which comprises

(a) subjecting a compound of the formula:

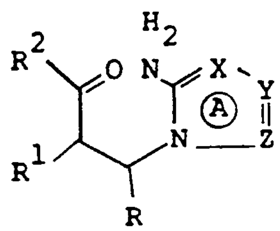

to dehydration condensation,

(b) subjecting a compound of the formula:

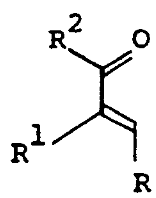

to condensation with a compound of the formula:

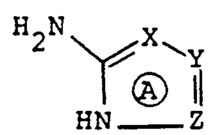

(c) subjecting a compound of the formula:

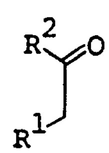

to dehydration condensation with a compound of the formula: R—CHO, and then subjecting the compound obtained to condensation with a compound of the formula:

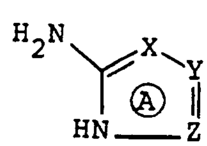

(d) reacting a compound of the formula:

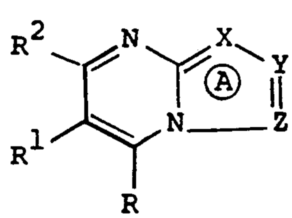

with hydrogen gas or a complex metal hydride, or

(e) if desired, converting the compound obtained into a pharmaceutically acceptable acid addition salt or a metal salt, wherein in the above formulae, each symbol is as defined in claim 1.

10. A pharmaceutical composition for the prophylaxis and treatment of circulatory diseases comprising a therapeutically effective amount of the compound of claim 1 and a pharmaceutically acceptable carrier thereof.

**Patentansprüche**

1. Polyazaheterocyclus-Derivat der Formel

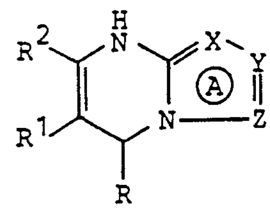

oder ein pharmazeutisch annehmbares Salz desselben, worin

R ein Wasserstoff-Atom, eine $C_{1-5}$-Alkyl-Gruppe, eine $C_{3-6}$-Cycloalkyl-Gruppe, eine Furyl-Gruppe, eine Thienyl-Gruppe, eine Pyridyl-Gruppe oder eine Phenyl-Gruppe ist, die gegebenenfalls durch einen oder zwei Substituenten, ausgewählt aus der aus einem Halogen-Atom, einer $C_{1-4}$-Alkyl-Gruppe, einer Trifluoromethyl-Gruppe, einer $C_{1-4}$-Alkoxy-Gruppe, einer Phenyl-$C_{1-3}$-alkyloxy-Gruppe, einer $C_{1-4}$-Alkylthio-Gruppe, einer Phenyl-$C_{1-3}$-alkylthio-Gruppe, einer $C_{2-4}$-Alkanoyl-Gruppe, einer Benzoyl-Gruppe, einer Hydroxyl-Gruppe, einer Amino-Gruppe, einer Nitro-Gruppe und einer Cyano-Gruppe bestehenden Gruppe, substituiert sein kann;

$R^1$ ein Wasserstoff-Atom; eine Cyano-Gruppe; eine Carbamoyl-Gruppe; eine Carboxyl-Gruppe; eine $C_{1-4}$-Alkoxycarbonyl-Gruppe, die gegebenenfalls in der Alkoxy-Struktureinheit durch einen oder zwei Substituenten substituiert sein kann, die ausgewählt sind aus der aus einer $C_{1-4}$-Alkoxy-Gruppe, einer Cyano-Gruppe, einer durch die Formel —$N(R^1_a)(R^1_b)$ bezeichneten Gruppe {worin jeder der Substituenten $R^1_a$ und $R^1_b$ eine $C_{1-4}$-Alkyl-Gruppe oder eine Phenyl-$C_{1-3}$-alkyl-Gruppe ist oder $R^1_a$ und $R^1_b$ zusammen mit dem benachbarten Stickstoff-Atom einen 5- bis 7-gliedrigen Heterocyclus bilden, der aus der aus Pyrrolidin, Piperidin, Morpholin, Piperazin, N-Methylpiperazin, N-(2-Hydroxyethyl)piperazin, Azepin und Diazepin bestehenden Gruppe ausgewählt ist}, einer Nitro-Gruppe, einer Phenyl-Gruppe und einer aus der aus Thienyl, Furyl und Pyridyl bestehenden Gruppe ausgewählten Heteroaryl-Gruppe bestehenden Gruppe; oder eine $C_{2-6}$-Alkanoyl-Gruppe, die aus der Acetyl, Propionyl, Butyryl, Isobutyryl, Valeryl, Isovaleryl, Pivaloyl und Hexanoyl bestehenden Gruppe ausgewählt ist;

$R^2$ eine $C_{1-5}$-Alkyl-Gruppe, eine Hydroxy-$C_{1-4}$-alkyl-Gruppe, eine Acetoxy-$C_{1-4}$-alkyl-Gruppe, eine Halogeno-$C_{1-4}$-alkyl-Gruppe, eine Dihalogeno-$C_{1-4}$-alkyl-Gruppe, eine Trifluoromethyl-Gruppe, eine $C_{1-4}$-Alkoxy-$C_{1-4}$-alkyl-Gruppe, eine Di-$C_{1-4}$-alkoxymethyl-Gruppe, eine $C_{1-4}$-Alkylcarbamoyloxy-$C_{1-4}$-alkyl-Gruppe, eine $C_{1-4}$-Alkylthiocarbamoyloxy-$C_{1-4}$-alkyl-Gruppe, eine Formyl-Gruppe, eine Hydroxyiminomethyl-Gruppe oder eine Cyano-Gruppe ist; und

X, Y und Z jeweils Glieder des Ringes A sind und N oder CH bezeichnen.

2. Verbindung nach Anspruch 1, bezeichnet durch die Formel

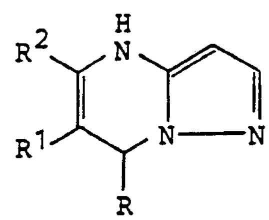

worin jedes Symbol die in Anspruch 1 angegebenen Bedeutungen hat, oder ein pharmazeutisch annehmbares Salz desselben.

3. Verbindung nach Anspruch 1, bezeichnet durch die Formel

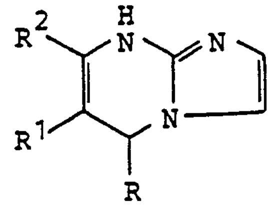

worin jedes Symbol die in Anspruch 1 angegebenen Bedeutungen hat, oder ein pharmazeutisch annehmbares Salz desselben.

4. Verbindung nach Anspruch 1, bezeichnet durch die Formel

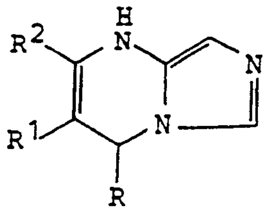

worin jedes Symbol die in Anspruch 1 angegebenen Bedeutungen hat, oder ein pharmazeutisch annehmbares Salz desselben.

5. Verbindung nach Anspruch 1: 1-Phenyl-2-piperidinoethyl-5-methyl-7-(3-nitrophenyl)-4,7-dihydropyrazolo[1,5-a]pyrimidin-6-carboxylat und dessen Diastereomer.

6. Verbindung nach Anspruch 1: 6-Ethoxycarbonyl-5-methyl-7-(2-trifluoromethylphenyl)-4,7-dihydropyrazolo(1,5-a]pyrimidin.

7. Verbindung nach Anspruch 1: 1-Phenyl-2-piperidinoethyl-7-methyl-5-(3-nitrophenyl)-5,8-dihydroimidazo[1,2-a]pyrimidin-6-carboxylat und dessen Diastereomer.

8. Verbindung nach Anspruch 1: 6-Ethoxycarbonyl-5,7-dimethyl-5,8-dihydroimidazo[1,2-a]pyrimidin.

9. Verfahren zur Herstellung eines Polyazaheterocyclus-Derivats der Formel

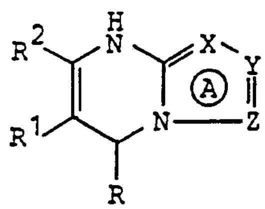

oder eines pharmazeutisch annehmbaren Salzes desselben, umfassend

(a) die Durchführung einer Dehydratisierungs-Kondensation einer Verbindung der Formel

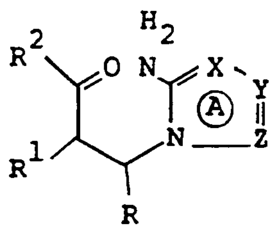

(b) die Durchführung einer Kondensation einer Verbindung der Formel

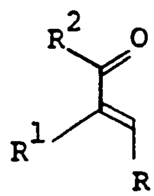

mit einer Verbindung der Formel

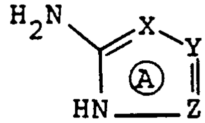

(c) die Durchführung einer Dehydratisierungs-Kondensation einer Verbindung der Formel

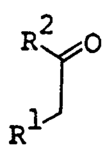

mit einer Verbindung der Formel R—CHO und danach die Durchführung einer Kondensation der erhaltenen Verbindung mit einer Verbindung der Formel

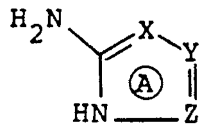

(d) die Umsetzung einer Verbindung der Formel

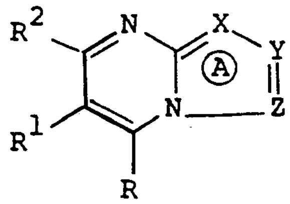

mit Wasserstoff-Gas oder einem komplexen Metallhydrid, oder

(e) gewünschtenfalls die Umwandlung der erhaltenen Verbindung in ein pharmazeutisch annehmbares Säureadditionssalz oder ein Metallsalz,

wobei in den vorstehenden Formeln jedes Symbol die in Anspruch 1 angegebenen Bedeutungen hat.

10. Pharmazeutische Zusammensetzung zur Prophylaxe und Behandlung von Kreislauf-Erkrankungen, umfassend eine therapeutisch wirksame Menge der Verbindung nach Anspruch 1 und einen pharmazeutisch annehmbaren Träger derselben.

**Revendications**

1. Dérivé polyazehétérocyclique ayant la formule:

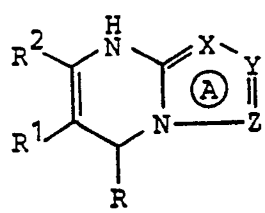

ou un sel pharmaceutiquement acceptable de celui-ci, formule dans laquelle

R est un atome d'hydrogène, un groupe alcoyl en $C_1$—$C_5$, un groupe cycloalcoyl en $C_3$—$C_6$, un groupe furyl, un groupe thiényl, un groupe pyridyl ou un groupe phényl qui peut être éventuellement substitué par un ou deux substituants choisis dans le groupe constitué par un atome d'halogène, un groupe alcoyl en $C_1$—$C_4$, un groupe trifluorométhyl, un groupe alcoxy en $C_1$—$C_4$, un groupe phényl-alcoyl (en $C_1$—$C_4$)-oxy un groupe alcoyl (en $C_1$—$C_4$)-thio, un groupe phényl-alcoyl (en $C_1$—$C_3$)-thio, un groupe alcanoyl en $C_2$—$C_4$, un groupe benzoyl, un groupe hydroxyl, un groupe amino, un groupe nitro et un groupe cyano;

$R^1$ est un atome d'hydrogène; un groupe cyano; un groupe carbamoyl; un groupe carboxyl; un groupe alcoxy (en $C_1$—$C_4$)-carbonyl qui peut être éventuellement substitué dans sa partie alcoxy par un ou deux substituants choisis dans le groupe consitué par un groupe alcoxy en $C_1$—$C_4$, un groupe cyano, un groupe représenté par la formule —N($Ra^1$)($Rb^1$) (dans laquelle chacun des $Ra^1$ et $Rb^1$ est un groupe alcoyl en $C_1$—$C_4$ ou un groupe phényl-alcoyl en $C_1$—$C_3$, ou bien $Ra^1$ et $Rb^1$ ensemble avec l'atome d'azote adjacent forment un hétérocycle ayant 5- à 7- éléments, choisi dans le groupe constitué par la pyrrolidine, la pipéridine, la morpholine, la pipérazine, la N-méthyl-pipérazine, la N-(2-hydroxyéthyl)pipérazine, l'azépine et la diazépine), un groupe nitro, un groupe phényl et un groupe hétéroaryl choisi dans le groupe constitué par thiényl, furyl et pyridyl; ou un groupe alcanoyl en $C_2$—$C_6$ choisi dans le groupe constitué par acétyl, propionyl, butyryl, isobutyryl, valéryl, isovaléryl, pivaloyl et hexanoyl;

$R^2$ est un groupe alcoyl en $C_1$—$C_5$, un groupe hydroxy-alcoyl en $C_1$—$C_4$, un groupe acétoxy-alcoyl en $C_1$—$C_4$, un groupe halo-alcoyl en $C_1$—$C_4$, un groupe dihalo alcoyl en $C_1$—$C_4$, un groupe trifluorométhyl, un groupe alcoxy (en $C_1$—$C_4$)-alcoyl (en $C_1$—$C_4$), un groupe di-alcoxy (en $C_1$—$C_4$)-méthyl, un groupe alcoyl (en $C_1$—$C_4$)-carbamoyloxy-alcoyl (en $C_1$—$C_4$), un groupe alcoyl (en $C_1$—$C_4$)-thiocarbamoyloxy-alcoyl (en $C_1$—$C_4$), un groupe formyl, un groupe hydroximinométhyl ou un groupe cyano; et

chacun des X, Y et Z est un membre de l'eanneau A et représente N ou CH.

2. Le composé de la revendication 1 représenté par la formule:

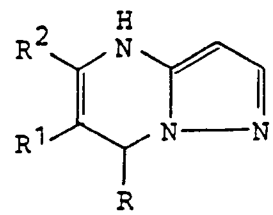

dans laquelle chaque symbole est tel que défini dans la revendication 1, ou un sel pharmaceutiquement acceptable de celui-ci.

3. Le composé de la revendication 1 représenté par la formule:

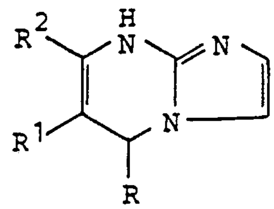

dans laquelle chaque symbole est tel que défini dans la revendication 1, ou un sel pharmaceutiquement acceptable de celui-ci.

4. Le composé de la revendication 1 représenté par la formule:

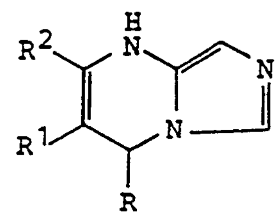

dans laquelle chaque symbole est tel que défini dans la revendication 1, ou un sel pharmaceutiqument acceptable de celui-ci.

5. Le composé de la revendication 1: 1-phényl-2-pipéridinoéthyl 5-méthyl-7-(3-nitrophényl)-4, 7-dihydropyrazolo[1,5-a]pyrimidine-6-carboxylate et le diastéréomère de celui-ci.

6. Le composé de la revendication 1: 6-éthoxycarbonyl-5-méthyl-7-(2-trifluorométhylphényl)-4,7-dihydropyrazolo[1,5-a]pyrimidine.

7. Le composé de la formule: 1-phényl-2-pipéridinoéthyl 7-méthyl-5-(3-nitrophényl)-5,8-dihydroimidazo[1,2-a]pyrimidine-6-carboxylate et le diastéréomère de celui-ci.

8. Le composé de la revendication 1: 6-éthoxycarbonyl-5,7-diméthyl-5,8-dihydroimidazo[1,2-a]-pyrimidine.

9. Un procédé pour préparer un dérivé polyazahétérocyclique de formule:

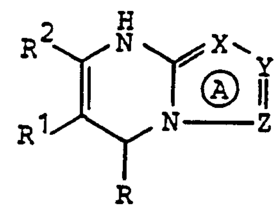

ou un sel pharmaceutiquement acceptable de celui-ci, qui comprend

a) ou soumet un composé de formule:

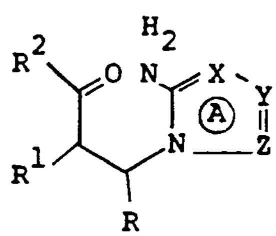

à une condensation avec déshydration,

b) ou soumet un composé de formule:

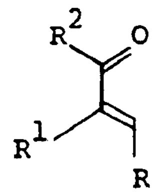

à une condensation avec un composé de formule:

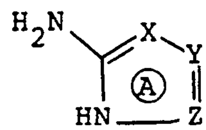

c) on soumet un composé de formule:

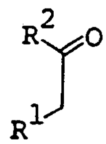

à une condensation avec déshydratation avec un composé de formule: R—CHO, et on soumet ensuite le composé obtenu à une condensation avec un composé de formule:

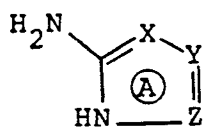

d) on fait réagir un composé de formule:

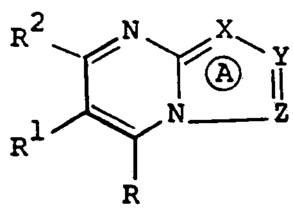

avec de l'hydrogène gazeux ou un hydrure métallique complexe, ou

e) si on le désire, on convertit le composé obtenu en un sel d'addition acide ou en sel métallique pharmaceutiquement acceptable, chaque symbole des formules ci-dessues étant tel que défini dans la revendication 1.

10. Une composition pharmaceutique pour la prophylaxie et le traitement des maladies de la circulation sanguine comprenant une quantité thérapeutiqment efficace du composé selon la revendication 1 et un véhicule, pharmaceutiquement acceptable, de celui-ci.